# EUROPEAN PATENT APPLICATION

(11) **EP 4 039 823 A1**
(43) Date of publication of application: **10.08.2022**
(21) Application number: 21155857.2
(22) Date of filing: 08.02.2021
(51) Int. Cl.: C12Q 1/6862

(54) **METHOD FOR MICRO-VOLUME, ONE-POT, HIGH-THROUGHPUT PROTEIN PRODUCTION**

(71) Applicant: Norwegian University of Science and Technology, by NTNU Technology Transfer AS, 7051 Trondheim (NO)
(72) Inventor: Kabisch, Johannes, 64367 Mühltal (DE); Schlichting, Niels, 64283 Darmstadt (DE); Zibulski, Darius, 64293 Darmstadt (DE)
(74) Representative: Fuchs Patentanwälte Partnerschaft mbB

(57) **Abstract**

The present invention pertains to a novel method for a micro-volume, one-pot, high-throughput protein production comprising the steps of providing DNA-starting-material; performing a ligase cycling reaction (LCR); performing a rolling-circle amplification (RCA); and performing a cell-free protein synthesis (CFPS) wherein all reactions take place in the same reaction container at a volume of 5 µl or less.

## Description

### Field of the Invention

The present invention pertains to a novel method for a micro-volume, one-pot, high-throughput protein production comprising the steps of providing DNA-starting-material; performing a ligase cycling reaction (LCR); performing a rolling-circle amplification (RCA); and performing a cell-free protein synthesis (CFPS) wherein all reactions take place in the same reaction container at a volume of 5 µl or less.

### Background of the Invention

For academic and industrial life science research, large-scale DNA assembly methods and gene expression systems are essential tools for realizing numerous applications and gaining far-reaching knowledge.

In this context, high-throughput methods have a prominent position by allowing a large number of experiments in a short time.

For example, the expression analysis of recombinant proteins is a challenging step in any high-throughput protein production pipeline. Often multiple expression systems and a variety of expression construct designs are considered for the production of a protein of interest. There is a strong need to triage constructs rapidly and systematically.

The prior art describes semi-automated methods for the simultaneous purification and characterization of proteins expressed from multiple samples of expression cultures from the *E. coli baculovirus* expression vector system (BEVS), and mammalian transient expression systems.

However, the efficacy of high-throughput methods depends greatly on the miniaturization of the respective reaction(s) and the increase of speed depends on the reduction of steps and time-consuming interruptions, for example such necessary for media-changes or cleaning of intermediate products.

These two requirements alone, i.e. the reduction of reaction volume and the avoidance of interruptions, pose already a significant challenge to the development of high-throughput reaction systems:

For example the reduction of reaction volume worsens the surface-to-volume ratio, the handling of ingredients and the susceptibility to disruptive effects. A reaction, which would readily take place in a 1 ml or 50 µl-reaction container, may not work at all in volumes necessary for high-throughput-systems, such as 5 µl or less.

Furthermore, interruptions, for example such as necessary for cleaning intermediate products from side-products, or changes of reaction conditions (i.e. media) in order to meet the right conditions for the next production step, represent serious obstacles when developing a high-throughput system.

The present invention, however, overcomes said obstacles and presents a new method which allows high-throughput processes for a successive sequence of DNA assembly, selection, multiplication and in vitro gene expression in very small volumes and in a single-reaction container (i.e. a "one-pot"-solution).

As such the method presented hereinunder is robust against background noise, applicable even to small amounts of starting material, quick, highly parallelisable (therefore less expensive) and easy to use.

### Detailed Description

In one aspect the present invention pertains to a method for a micro-volume, one-pot, high-throughput protein production comprising the steps of:
a. providing DNA-starting-material;
b. performing a ligase chain reaction (LCR)
c. performing a rolling-circle amplification (RCA); and
d. performing a cell-free protein synthesis (CFPS);
wherein all reactions take place in the same reaction container at a volume of 5 µl or less, preferable 4 µl or less, even more preferable 3.6 µl or less.

In one aspect of the method the DNA-starting-material provided in step a.) comprises at least one linear DNA of between 0.25 nM to 3 nM starting material and at least one bridging oligo.

It is one of the important benefits of the described methods, that very little starting material of DNA may be used for reliable results, rendering it specifically useful for application in which only small amounts of DNA are available (e.g. in diagnosis and/or analytic scenarios).

Thus, at least one linear DNA-starting-material may be present in an amount of about 0.25 nM or more, of about 0.45 nM or more, of about 0.85 nM or more, of about 1 nM or more, of about 1.5 nM or more, and up to about 2 nM or less, up to about 2.5 nM or less, up to about 3 nM or less. In one aspect the DNA-starting-material may be present in an amount of between about 0.3 nM and about 2.5 nM, more preferred in an amount of between 0.35 nM and 2 nM, even more preferred in an amount of between about 0.4 nM and about 1.5 nM.

In another aspect of the invention the LCR-reaction of step b.) is performed in a LCR mixture comprising a suitable buffer.

Such a buffer may consist for example of about 20 mM Tris-HCI (pH 8.3), about 25 mM KCI, about 10 mM MgCl₂, about 0.5 mM NAD and about 0.01% Triton^{®} X-100. The skilled person will be aware that the buffer conditions need to be adapted to the specific ligase which is used.

The LCR-mixture further comprises about 0.05 to 2 U/µl, in one embodiment at least 0.1 U/µl, in one embodiment at least 0.2 U/µl, in one embodiment at least 0.3 U/µl, in other embodiments between about 0.1 U/µl and up to 1 U/µl, between about 0.2 U/µl, and up to 0.8 U/µl, between about 0.25 U/µl, and up to 0.75 U/µl, of at least one highly thermostable DNA ligase, for example a ligase from a thermophilic bacterium, such as for example *Thermus aquaticus, Thermus filiformis, Thermotoga maritima, Thermococcus barophilus* and/or a highly thermostable DNA ligase from a cyanobacterium. In certain embodiments also a combination of two or more highly thermostable DNA ligases may be used.

In another aspect of the invention the LCR mixture is subjected to the following LCR temperature program:
Step 1: between 90-98°C for between 1-3 min., in one embodiment at least 94°C for about 2 min; at least 90°C for about 3 min; at least 92°C for about 2.5 min; at least 96°C for about 1.5 min and/or at least 98°C for about 1 min.;
Step 2: between 90-98°C for between 5-30 s; in some embodiments at least 94°C for at least 10 s; at least 90°C for about 30 s; at least 92°C for about 20 s; at least 96°C for about 5 s and/or at least 98°C for about 3 s;
Step 3: between 60-70°C for between 45-180 s, in some embodiments at least 66°C for 90 s; at least 60°C for about 180 s; at least 62°C for about 120 s; at least 68°C for about 60 s and/or at least 70°C for about 45 s;
   and
   repeat the steps 2 and 3 for at least between 20 - 30 times; in some embodiments for at least 20 times, at least 22 times, at least 24 times; at least 26 times; and less than 34 times, less than 32 times, less than 30 times, less than 28 times; in some embodiments between 22 and 28 times.

It is one aspect of the present invention that significant less cycles are needed as compared to prior art systems, which use about 150 to 200 cycles, or even more.

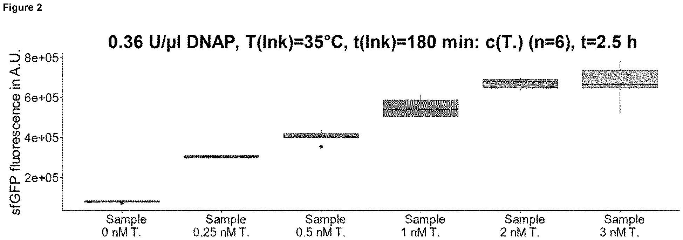

Due to the concept of the present method, fluctuations of LCR yield in the first step of the workflow have a comparatively lesser effect on the final protein yield in CFPS. This is manifested by the fact, that in the concentration range from about 0.25 nM to about 3 nM of the LCR reaction product used, each doubling of concentration leads to a constant signal increase (see also Figure 2).

This logarithmic relationship between LCR efficiency and product yield or product signal allows larger differences in LCR efficiency without relevant signal losses.

When starting the RCA with about 2 nM of LCR reaction product, the system reaches in some cases already saturation, which means that constant product yields can be achieved from this limit value. This also means that starting amounts of more than 3 nM are less preferred, since they do not result in higher yield.

Due to the concept of the present method, the RCA amplification levels out "noise effects" of the LCR and therefore allows sensitive detection of e.g. novel protein variants or genetics circuits without needing to worry about noise from varying DNA assembly efficiencies.

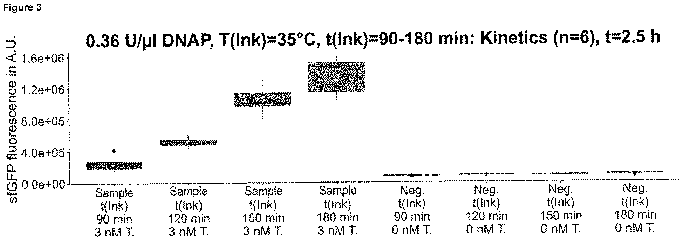

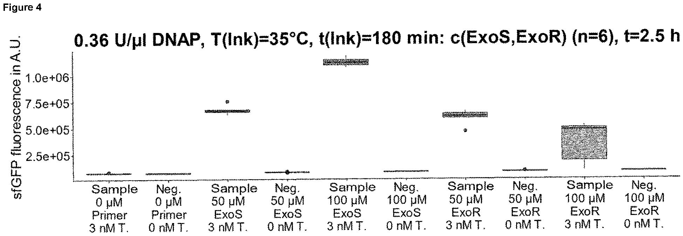

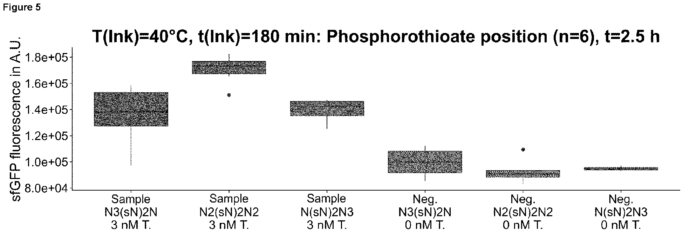

In another aspect of the invention the RCA-reaction of step c.) is performed according to the following steps:
Step 1: Adding an annealing mixture to the LCR-mixture, comprising between about 50 to about 200 µM random phosphorothioate-protected DNA hexamers, such as N₃(sN)₂N-phosphorothioate, N₂(sN)₂N₂-phosphorothioate and N(sN)₂N₃-phosphorothioate, preferably N₂(sN)₂N₂-phosphorothioate, and a buffer suitable for the specific RCA-DNA-polymerase, (for example a phi29 DNA polymerase reaction buffer comprising 50 mM Tris-HCI, 10 mM MgCI2, 10 mM (NH4)2SO4, 4 mM DTT, pH 7.5 @ 25°C);
   In further embodiments also from about 80 µM to about 150 µM random N₂(sN)₂N₂-phosphorothioate-protected DNA hexamers may be used, in one embodiment 100 µM random N₂(sN)₂N₂-phosphorothioate-protected DNA hexamers are used.
   As outlined above, the hexamers can be protected by incorporating phosphorothioate such as N₃(sN)₂N-phosphorothioate, N₂(sN)₂N₂-phosphorothioate and N(sN)₂N₃-phosphorothioate, wherein "N" means herein any random nucleotide, "sN" mean a protected (i.e. s = "stabilized") nucleotide by phosphorothioate and the lower case number depicts the number of nucleotides of each type within the hexamer. The denotation also shows the respective position of the normal and modified nucleotides within the hexamer, i.e. N₂(sN)₂N₂-phosphorothioate can be translated in: "N-N-sN-sN-N-N"'. It has been found that N₂(sN)₂N₂-phosphorothioate, i.e. the hexamer with the two protected nucleotides in the middle, shows the best signals (see Figure 5), whereas the hexamers with asymmetric and/or terminally protected nucleotides show also signals, but weaker. Thus, N₂(sN)₂N₂-phosphorothioate is the preferred protection for the hexamers.
   In other embodiments the protection can be made with other backbone-protecting agents, such as N(sN)₄N. And in some embodiments one of the following hexamer variants may be used, but less preferred are hexamers with asymmetric and/or terminally protected nucleotides, such as N₂(sN)N₃, N₃(sN)N₂, N(sNhN₂, N₂(sN)₃N, N(sN)N₄, N₄(sN)N, N₅(sN), N(SN)₂N₃, N₃(SN)₂N, N₄(SN)₂, N(SN)₃N₂, N₂(SN)₃N, N₃(sN)₃, N₂(sN)₄ or N(sN)₅.
   Although the method can also be performed without protected nucleotides, phosphorothioate (PS) backbone modification of the DNA hexamers seem to protect these from rapid degradation during the reaction, resulting in improved signal strength and, as a result, in an improved yield of product as well as improved signal-to-noise ratios.
   Both, the use of lower primer concentrations of less than about 50 µM and the use of exonuclease-resistant primers (ExoR) with 3'-terminal with phosphorothioat has negative effects on the signal strength and should be avoided (see Figure 4).
Step 2: Heating to between about 90-99°C for about 1-5 min, in one embodiment heating to about 95°C for about 3 min; then placing the mixture on ice;
   In further embodiments the heating time is between about 1 and about 5 minutes; in further embodiments between about 2 and about 4 minutes. Shorter heating times of less than about 1 minutes result in too low signal strengths, while longer heating times of more than 5 minutes, for example more than about 6 minutes, more than about 10 minutes, just delay the whole process without significant signal improvement. In fact, the chances of an undesired degradation of certain components may be increased by longer heating times and, thus, are less preferred.
Step 3: Adding an incubation mixture comprising a RCA-polymerase, for example comprising dNTPs, BSA, phi29 DNA polymerase, *E. coli* inorganic pyrophosphatase, DTT (dithiothreitol);
   In another embodiment the RCA polymerases is selected from the group comprising Φ29 (Phi29), Bst, and Vent exo-DNA polymerase and/or any combination thereof.
   It was found that organic *E. coli* pyrophosphatases do have a negative effect on the reaction.
   Since Φ29 DNA polymerase has the best processivity and strand displacement ability among all aforementioned polymerases, it is preferred in this invention. However, depending on the specific problem, also the other polymerase could be applied in other embodiments.
Step 4: Incubating for between about 120 to about 240 min at between about 30 to about 40°C; in one embodiment in the incubation is done for at least about 180 min at about 35°C. In some embodiments the incubation time may be as short as between 85 to about 100 min.
   In further embodiments the incubation time is between about 120 and about 240 minutes; in further embodiments between about 150 and about 210 minutes. Shorter incubation times of less than about 120 minutes, less than about 80 minutes, less than about 60 minutes result in too low signal strengths, while longer incubation times of more than about 240 minutes, for example more than about 300 minutes, more than about 360 minutes, more than about 420 minutes are unacceptable from a time point of view and unnecessary to achieve the desired concentrations (see Figure 3).
   Different from polymerase chain reaction (PCR), RCA can be conducted at a constant temperature (for example at room temperature of about 25°C, at about 30°C or up to about 37°C, in other embodiments at a temperature between about 4°C to about 45°C) in both free solution and on top of immobilized targets (solid phase amplification).
Step 5: Inactivating for between about 5 to about 20 min at between about 60 to about 70°C; in one embodiment inactivation is done for about 10 min at about 65°C;
   In further embodiments the inactivating time is between about 5 and about 20 minutes; in further embodiments between about 7 and about 15 minutes. Shorter heating times of less than about 5 minutes result in too low signal strengths, while longer heating times of more than about 20 minutes, for example more than about 25 minutes, more than about 30 minutes, just delay the whole process without significant signal improvement. In fact, the chances of an undesired degradation of certain components may be increased by longer heating times and, thus, are less preferred.
Step 6: Cooling the mixture to less than about 30°C, in one embodiment less than about 29°C, less than about 25°C, less than about 23°C. In one embodiment at about 20°C to about 23°C.

The application of the RCA-step allows not only the amplification of the DNA-LCR-product, but it also represents a "selection" step, since the RCA will only amplify correctly ligated and thus circular DNA-LCR-products.

DNA assemblies are usually only successful for few molecules. In state-of-the art techniques such assemblies are transformed into e.g. *E. coli bacteria* which selectively only replicate and thus amplify assembled replicons, but not single stranded DNA parts. The RCA described in this methods emulates this process by selectively amplifying only circularized DNA. Only if all LCR fragments have been successfully ligated a ring closure of the DNA occurs and the RCA-polymerase produces long concatemers of this DNA, which are required for successful CFPS.

Thus, the RCA-reaction serves both as amplification as well as a selection step. Through the intermediate use of multiply-primed RCA in the workflow, the generated LCR product can be amplified to such an extent that it enables a clearly detectable protein synthesis in the final *in vitro* CFPS. This is in contrast to all workflows in the prior art, which have focused on optimizing the LCR reaction conditions in order to achieve higher LCR product yields.

In addition, the system could be extended to other applications that focus on the generation of combinatorial libraries via LCR and the analysis of its gene products. This range of accessible gene products can be significantly expanded through the use of other prokaryotic and eukaryotic cell extracts and/or mixtures thereof.

In yet another aspect of the invention the CFPS-reaction of step d.) is performed according to the following steps:
Step 1: Adding a CFPS-mixture (for example consisting of 818 nl *E. coli* cell extract, which usually contain about 20 to about 30 mg/ml of proteins, and 982 nl cell extract buffer, consisting of magnesium-glutamate, potassium-glutamate, amino acid solution, energy solution (3.6 ml 2 M HEPES, 144 µl dest. H₂O, 1.39 ml Nucleotide Mix, 576 µl 50 mg/ml tRNA, 576 µl 65 mM CoA, 276 µl 175 mM NAD⁺, 170 µl 650 mM cAMP, 288 µl 33,9 mM folinic acid, 144 µl 1 M spermidine, 3.09 ml 1.4 M 3-PGA), DTT, PEG-8000, Mi-liQ-H₂O;
   In other embodiments the cell-extract may be derived from *Bacillus subtilis, Bacillus licheniformis, Paenibacillus polymyxae* and/or blends of different cell extracts originating from these organisms.
Step 2: Incubating the CFPS-mixture for at least between about 2 to about 4 hours at less than about 30°C in another embodiment for at least about 2.5 hours at about 29°C; in some embodiments for at least about 2 hours at about 29°C, at least about 3 hours at about 29°C, at least about 3.5 hours at about 29°C, and up to about 4 hours at about 29°C, up to about 4.5 hours at about 29°C, up to about 5 hours at about 29°C.
   and, optional,
Step 3: Harvesting the protein product.
   Suitable harvesting methods may be: Centrifugation followed by depth filtration, centrifugation followed by filter-aid enhanced depth filtration, microfiltration, immobilized metal affinity chromatography (IMAC), size-exclusion chromatography (gel filtration) and/or affinity tag purification.

In yet another aspect of the invention the method is performed in a 384-well-plate, i.e. in 384 simultaneous reactions. However, more preferred are 1536-well-plates or even 3456-well plates. Thus, 1536 or 3456 simultaneous reactions can be performed in one turn resulting in a suitability of the inventive method in ultra-high-throughput-screening (Ultra HTS or uHTS) applications.

Such a high-throughput screening is used in particular to search for new, biologically active substances from which lead structures can be derived in order to develop new drugs.

The inventive method can be performed in less than about 20 hours, less than about 15 hours, less than about 10 hours, about 7 hours, in some embodiments even in less than about 6 hours.

Time consuming steps are the ligation cycles, which in total need about 1 hour, the RCA-reaction of about 3 hours and the CFPS-reaction of about 2 hours. Thus, the entire method workflow including protein production is finished within about 6-7 hrs. In relation to pure reaction, incubation and measurement times requires only a third of the time of conventional prior art workflows with about 18 to about 23 hrs.

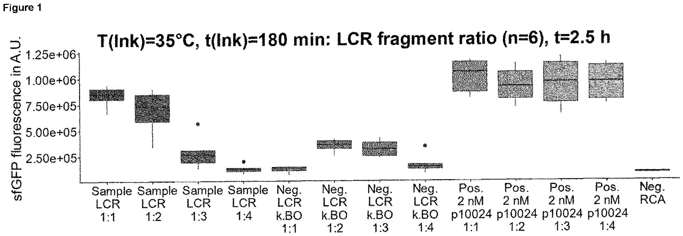

The design of the method of the present invention as a high-throughput, one-pot method allows the simultaneous measurement of several samples (see Figure 1) with a high statistical relevance (n=6 repetitions per sample). The workflow can be partially automated and further accelerated by using nanoliter dispensers, such as for example the I-DOT^{®} or the LabCyte^{®} ECHO (autodispenser), to mix the reaction mixtures.

For performing the inventive method a hydro-cycling system may be more suitable than a thermocycler.

Since the expression takes place in a cell-free (CFPS) system, also proteins otherwise toxic for cells may be expressed.

Furthermore, due to the ultra-high-throughput-screening abilities, the described method can be also used in order to improve existing proteins, enzymes, antibodies, vaccines and the like by combining the described method with *in vitro* evolution and/or selection processes.

### Definitions

The term *"between"* as used herein, encompasses also the exact value which is denoted, thus, *"between 5 -* 10" also includes the exact values 5 and 10, and any value in-between.

The term "about" as used herein, encompasses also values of ± 10% of the value denoted herein. For example "about 10" encompasses also "9" and "11", and any value in-between.

The term "*in-vitro*-polymerase-mediated rolling-circle amplification (RCA)" as used herein, describes a process of unidirectional nucleic acid replication that can rapidly synthesize multiple copies of circular molecules of DNA or RNA, such as plasmids, the chromosomes of bacterio-phages, and the circular RNA chromosome of viroids. Some eukaryotic viruses also replicate their DNA or RNA via the rolling circle mechanism.

There are typically the following steps involved in a DNA-RCA reaction:
- Circular template ligation using special DNA ligases.
- Primer-induced single-strand DNA elongation. Multiple primers can be employed to hybridize with the same circle. As a result, multiple amplification events can be initiated, producing multiple RCA products ("multi-primed RCA"). A linear RCA product can be converted into multiple circles using restriction enzyme digestion followed by template mediated enzymatic ligation.
- (Optional) Amplification product detection and visualization, which is most commonly conducted through fluorescent detection, with fluorophore-conjugated dNTP, fluorophore-tethered complementary or fluorescently-labeled molecular beacons. In addition to the fluorescent approaches, gel electrophoresis is also widely used for the detection of RCA product.

The term "cell-free protein synthesis (CPFS)" as used herein refers to the production of protein using biological machinery in a cell-free system, that is, without the use of living cells. The *in vitro* protein synthesis environment is not constrained by a cell wall or homeostasis conditions necessary to maintain cell viability.

Thus, CFPS enables direct access and control of the translation environment which is advantageous for a number of applications including co-translational solubilization of membrane proteins, optimization of protein production, incorporation of non-natural amino acids, selective and site-specific labelling.

Due to the open nature of the system, different expression conditions such as pH, redox potentials, temperatures, and chaperones can be screened. Since there is no need to maintain cell viability, toxic proteins can be produced.

CFPS has many advantages over the traditional *in vivo* synthesis of proteins. Most notably, a cell-free reaction, including extract preparation, usually takes few hours and up to 1 -2 days, whereas *in vivo* protein synthesis may take 1-2 weeks.

CFPS is an open reaction. The lack of cell wall allows direct manipulation of the chemical environment. Samples are easily taken, concentrations optimized, and the reaction can be monitored. In contrast, once DNA is inserted into live cells, the reaction cannot be accessed until it is over and the cells are lysed.

Another advantage to CFPS is the lack of concern for toxicity. Some desired proteins and labeled proteins are toxic to cells when synthesized. Since live cells are not being used, the toxicity of the product protein is not a significant concern.

These advantages enable numerous applications.

A major application of CFPS is incorporation of unnatural amino acids into protein structures. The openness of the reaction is ideal for inserting the modified tRNAs and unnatural amino acids required for such a reaction.

Synthetic biology has many other uses in fields such as protein evolution, nanomachines, nucleic acid circuits, and synthesis of virus-like particles for vaccines and drug therapy.

Thus, in one aspect the present invention pertains to a method for a micro-volume, one-pot, high-throughput protein production comprising the steps of:
a. providing DNA-starting-material;
b. performing a ligase chain reaction (LCR);
c. performing a rolling-circle amplification (RCA); and
d. performing a cell-free protein synthesis (CFPS);
wherein all reactions take place in the same reaction container at a volume of 5 µl or less.

In a further aspect the present invention pertains to the method, wherein the DNA-starting-material provided in step a.) comprises at least one vector, at least one insert and at least one bridging oligo.

In yet a further aspect the present invention pertains to the method, wherein the LCR-reaction of step b.) is performed in a LCR mixture comprising buffer suitable for the performance of the highly thermostable DNA ligase and comprising between 0.2 - 2 U/µl, in one embodiment about 0.2 U/µl, of at least one highly thermostable DNA ligase.

In yet a further aspect the present invention pertains to the method, wherein the LCR mixture is subjected to the following LCR temperature program:
Step 1: between 90-98°C for between 1-3 min;
Step 2: between 90-98°C for between 5-30 s;
Step 3: between 60-70°C for between 45-180 s;
and repeating the steps 2 and 3 for at least between 20 and 34 times.

In yet a further aspect the present invention pertains to the method, wherein the RCA-reaction of step c.) is performed according to the following steps:
Step 1: Adding an annealing mixture to the LCR-mixture, comprising between 50 - 200 µM phosphorothioate-protected random DNA hexamers selected from N₃(sN)₂N-phosphorothioate, N₂(sN)₂N₂-phosphorothioate and N(sN)₂N₃-phosphorothioate, and a RCA-DNA polymerase reaction buffer;
Step 2: Heating to between 90-99°C for 1-5 min; then placing the mixture on ice;
Step 3: Adding an incubation mixture, comprising a RCA-DNA-polymerase;
Step 4: Incubating for between 120-240 min at between 30-40°C;
Step 5: Inactivating for between 5-20 min at between 60-70°C:
Step 6: Cooling the mixture below 30°C.

In yet a further aspect the present invention pertains to the method, wherein the CFPS-reaction of step d.) is performed according to the following steps:
Step 1: Adding a CFPS-reaction-mixture;
Step 2: Incubating the CFPS-reaction-mixture for at least between 2 - 5, in one embodiment between 2 - 4.5, hours at less than 30°C;
   and, optional,
Step 3: Harvesting the protein product.

In yet a further aspect the present invention pertains to the method, wherein the method is performed up to 384-times simultaneously in a 384-microwell-plate; up to 1536-times simultaneously in a 1536-microwell-plate; or up to 3456-times in a 3456-microwell-plate.

In yet a further aspect the present invention pertains to the method, wherein the method is performed in less than about 7 hours.

In yet another aspect the present invention pertains to the production of a nucleic acid and/or a protein by said method(s).

In one embodiment the nucleic acid and/or protein produced by the inventive method may be used as a medicament, e.g. a therapeutic antibody, a vaccine, a peptidic drug, an enzyme, a protein drug (e.g. insulin), or the like.

In yet another aspect the present invention pertains to the use of said method(s) for screening a drug, producing and/or improving a nucleic acid and/or an enzyme, a polypeptide or a binding protein.

In yet another aspect the present invention pertains to the use of said method(s) for diagnostic purposes, such as for example the amplification of biomarkers within a sample.

### Short description of the figures

- Figure 1: In order to test the ideal vector-to-insert ratio, sfGFP fluorescence (extinction: 470 nm, emission: 515 nm) (after 2.5 h *in vitro* CFPS) of the following samples was compared (in 6-fold repetition) in the inventive workflow: LCR samples with a three-part plasmid coding for sfGFP with a vector-to-insert ratio of 1:1, 1:2, 1:3 and 1:4 (2 nM vector in each sample), LCR negative control (Neg. LCR) without bridging oligos (k.BO)), LCR positive control (Pos.) with 2 nM of the resulting plasmid p10024 instead of vector and insert, and a RCA negative control (Neg. RCA) without template DNA. The LCR mixtures were filled manually in the wells, while all other reagents were added by the I-DOT. The samples 1 and 2 with a vector-to-insert ratio of 1:1 and 1:2, respectively, showed a similar signal as the positive control.
- Figure 2: In order to test the ideal amounts of starting material, the sfGFP fluorescence (extinction: 470 nm, emission: 515 nm) (after 2.5 h *in vitro* CFPS) of the following samples (in 6-fold repetition) was tested in the inventive workflow: 0 nM, 0.25 nM, 0.5 nM, 1 nM, 2 nM and 3 nM of plasmid p10024 (sample) instead of the LCR reaction mixture. All reagents were added to the reaction vessels (wells) via the I-DOT. A clear signal can already be observed at amounts of just 0.25 nM of starting material. At amounts of 2 nM and more a saturation became visible such that amounts of 3 nM or more did not result in significant increase in signal.
- Figure 3: In order to find the best incubation times for the RCA, sfGFP fluorescence (extinction: 470 nm, emission: 515 nm) (after 2.5 h *in vitro* CFPS) of the following samples (in 6-fold repetition) was tested in the inventive workflow: 3 nM of plasmid p10024 (sample) instead of the LCR reaction approach with 90 min, 120 min, 150 min and 180 min RCA incubation time and the corresponding negative controls (Neg.) with 0 nM of plasmid p10024. It turned out that incubation times of about 180 min resulted in the best signals.
- Figure 4: In order to test the influence of exonuclease sensitive (ExoS) or exonuclease resistant (ExoR) primers on the reaction, sfGFP fluorescence (extinction: 470 nm, emission: 515 nm) (after 2.5 h *in vitro* CFPS) of the following samples (in 6-fold repetition) was tested in the inventive workflow: 3 nM of plasmid p10024 (sample) instead of the LCR reaction approach with 0 µM, 50 µM and 100 µM exonuclease sensitive (ExoS) or exonuclease resistant (ExoR) primers and the corresponding negative controls (Neg.) with 0 nM of plasmid p10024. It turned out that exonuclease resistant (ExoR) primers with 3' terminal phosphorothioates did not positively alter the overall reaction performance, in fact exonuclease resistant (ExoR) primers with 3' terminal phosphorothioates resulted in slightly weaker signals and should be avoided.
- Figure 5: In order to test other protective approaches, sfGFP fluorescence of the samples was tested with N₃(sN)₂N, N₂(sN)₂N₂ and N(sN)₂N₃ primers (template / T .: 3 nM p10024) and the negative controls / Neg. with N₃(sN)₂N, N₂(sN)₂N₂ and N(sN)₂N₃ primers (template / T .: 0 nM p10024) in 6-fold repetition (n = 6) after 180 min RCA at 40°C (EquiPhi29 DNA polymerase) and 2.5 h CFPS. It turned out that modification with N₃(sN)₂N, N₂(sN)₂N₂ and N(sN)₂N₃ primers did positively alter the overall reaction performance, and resulted in stronger signals. N₂(sN)₂N₂ showed the best signal and was used for further experiments.

### Examples

### 1. DNA acquisition and preparation of DNA constructs.

DNA for the assembly reaction can be obtained by all means resulting in linear DNA fragments such as PCR, DNA synthesis or using site specific nucleases. Using minimal amounts of template for PCR (i.e. 0.1 pM) greatly reduces carry over of template DNA which, if the template DNA is circular, results in RCA of the template DNA.

Carry over can be further reduced by digestion with a restriction enzyme, in this example Dpnl was used.

Any DNA used in the assembly reaction must be phosphorylated at the 5'-end before the LCR reaction. This can be for example achieved by using phosphorylated oligonucleotides for the PCR or by phosphorylating the DNA fragments before LCR. Designing bridging oligos to have a melting temperature of 67.8°C for each annealing-half using the "SantaLucia (1998)" algorithm with salt-correction greatly improves efficiencies.

Alternatively, other calculation algorithms may be used for generating the bridging oligos, such as published in *"*Optimization of the experimental parameters of the ligase cycling reaction", Niels Schlichting, Felix Reinhardt, Sven Jaeger, Michael Schmidt, Johannes Kabisch, Synthetic Biology, Volume 4, Issue 1, 2019, ysz020, https://doi.org/10.1093/synbio/ysz020.

### 2. Micro-volume LCR-reaction.

In the first step, the ligase-chain reaction (LCR) is carried out, whose reaction mixture consists of 2 nM vector, 2 nM of each insert, 30 nM bridging oligos, 1x buffer for a highly thermostable DNA ligase (20 mM Tris-HCI (pH 8.3), 25 mM KCI, 10 mM MgCI2, 0.5 mM NAD, and 0.01% Triton^{®} X-100) and 0.3 U/µl highly thermostable DNA ligase. An autodispenser is used to dispense 0.612 µl of LCR reaction mixture into the wells of a low volume 384-well plates or preferebly a 1536-well plate sealed with an aluminum tape. Those 0.612 µl LCR mixture per well are subjected to the following LCR temperature program:
1. 94°C for 2 min,
2. 94°C for 10 s,
3. 66°C for 90 s; and
4. repeat the steps 2 and 3 for at least 24 times.

### 3. Rolling-circle amplification of synthetic DNA fragments and plasmids.

Multiply-primed RCA takes place by adding 468 nl from the annealing mixture (consisting of 385 µM random N₂(sN)₂N₂-phosphorothioate-protected DNA hexamers and the 3.85x phi29 DNA Polymerase Reaction Buffer comprising 50 mM Tris-HCI, 10 mM MgCl₂, 10 mM (NH₄)2SO₄, 4 mM DTT, pH 7.5 @ 25°C) to the 0.612 µl LCR mixture via an autodispenser.

The resulting 1.08 µl annealing RCA mixture (166 µM random N₂(sN)₂N₂-phosphorothioate-protected DNA hexamers, 1.66x phi29 buffer) per well are covered with an aluminum tape, heated to 95°C for 3 min and then placed on ice to enable annealing of the random hexamers to the LCR product. Samples should be stored on ice at least 5 minutes before the next step.

Then, 0.72 µl from the incubation mixture (consisting of 2.5 nM of each dNTP, 1 mg/ml BSA, 0.9 U/µl phi29 DNA polymerase, 0.01 U/µl *E*. *coli* pyrophosphatase, 10 mM dithiothreitol (DTT)) are dispensed to the 1.08 µl with an autodispenser.

That 1.8 µl incubation RCA mixture (612 nl LCR mixture, 100 µM N₂(sN)₂N₂-phosphorothioate-protected random DNA hexamers, 1 mM of each dNTP, 0.4 µg/µl BSA, 1x phi29 buffer, 0.36 U/µl phi29 DNA polymerase, 0.004 U/µl *E. coli* pyrophosphatase, 4 mM DTT, MilliQ^{®} H₂O) is incubated for 180 min at 35°C followed by inactivation for 10 min at 65°C.

### 4. Cell-free protein production.

In the third step, the cell extract-based *in vitro* CFPS is carried out by first dispensing 1.8 µl CFPS-mixture (consisting of 818 nl *E.coli* cell extract and 982 nl cell extract buffer, consisting of 94.26 µl 500 mM magnesium-L-glutamate, 251.37 µl 3000 mM potassiun-L-glutamat, 531.56 µl Amino acid solution (solution contains 1.5 ml with 168 mM of each of the amino acids Ala, Arg, Asn, Asp, Gin, Glu, Gly, His, Ile, Lys, Met, Phe, Pro, Ser, Thr, Val, Trp, Tyr, Cys and 1,5 ml 140 mM Leu, 12 ml dest. H₂O), 336.66 µl Energy solution (consists of 3,6 ml 2 M HEPES, 144 µl dest. H₂O, 1,39 ml Nucleotide Mix, 576 µl 50 mg/ml tRNA, 576 µl 65 mM CoA, 276 µl 175 mM NAD + , 170 µl 650 mM cAMP, 288 µl 33.9 mM folinic acid, 144 µl 1 M spermidine, 3.09 ml 1.4 M 3-PGA.), 70,70 µl 100 mM DTT, 235.66 µl 40% (w/v) PEG-8000, 459.33 µl MiliQ-H2O) to the cooled 1.8 µl incubation RCA mixture using an autodispenser.

Finally, that 3.6 µl CFPS-mixture is incubated for at least 2.5 hours at 29°C so that the final protein synthesis can proceed.

## Claims

1. Method for a micro-volume, one-pot, high-throughput protein production comprising the steps of:
a. providing DNA-starting-material;
b. performing a ligase chain reaction (LCR);
c. performing a rolling-circle amplification (RCA); and
d. performing a cell-free protein synthesis (CFPS);
wherein all reactions take place in the same reaction container at a volume of 5 µl or less.

2. The method of claim 1, wherein the DNA-starting-material provided in step a.) comprises at least one vector, at least one insert and at least one bridging oligo.

3. The method of claim 1 or 2, wherein the LCR-reaction of step b.) is performed in a LCR mixture comprising buffer suitable for the performance of the highly thermostable DNA ligase and comprising between 0.2 - 2 U/µl of at least one highly thermostable DNA ligase.

4. The method of claim 3, wherein the LCR mixture is subjected to the following LCR temperature program:
Step 1: between 90-98°C for between 1-3 min;
Step 2: between 90-98°C for between 5-30 s;
Step 3: between 60-70°C for between 45-180 s;
and repeating the steps 2 and 3 for at least between 20 and 34 times.

5. The method of any of the previous claims, wherein the RCA-reaction of step c.) is performed according to the following steps:
Step 1: Adding an annealing mixture to the LCR-mixture, comprising between 50 - 200 µM random phosphorothioate-protected DNA hexamers, selected from N₃(sN)₂N-phosphorothioate, N₂(sN)₂N₂-phosphorothioate and N(sN)₂N₃-phosphorothioate, and a RCA-DNA polymerase reaction buffer;
Step 2: Heating to between 90-99°C for 1-5 min; then placing the mixture on ice;
Step 3: Adding an incubation mixture, comprising a RCA-DNA-polymerase;
Step 4: Incubating for between 120-240 min at between 30-40°C;
Step 5: Inactivating for between 5-20 min at between 60-70°C:
Step 6: Cooling the mixture below 30°C.

6. The method of any of the previous claims, wherein the CFPS-reaction of step d.) is performed according to the following steps:
Step 1: Adding a CFPS-reaction-mixture;
Step 2: Incubating the CFPS-reaction-mixture for at least between 2 - 5 hours at less than 30°C;
and, optional,
Step 3: Harvesting the protein product.

7. The method of any of the previous claims, wherein the method is performed up to 384-times simultaneously in a 384-microwell-plate; up to 1536-times simultaneously in a 1536-microwell-plate; or up to 3456-times in a 3456-microwell-plate.

8. The method of any of the previous claims, wherein the method is performed in less than 7 hours.

9. Nucleic acid produced by a method of any of the claims 1 to 8.

10. Protein produced by a method of any of the claims 1 to 8.

11. Nucleic acid of claim 9 and/or protein of claim 10 for the use as a medicament.

12. Use of the method of any of the claims 1 to 8 for screening a drug.

13. Use of the method of any of the claims 1 to 8 for producing and/or improving a nucleic acid.

14. Use of the method of any of the claims 1 to 8 for producing and/or improving an enzyme, a polypeptide or a binding protein.

15. Use of the method of any of the claims 1 to 8 for diagnosis.
